(19) Europäisches Patentamt
European Patent Office
Office européen des brevets

(11) Veröffentlichungsnummer: **0 084 516 B1**

(12) **EUROPÄISCHE PATENTSCHRIFT**

(45) Veröffentlichungstag der Patentschrift: **04.12.85**

(51) Int. Cl.⁴: **A 61 K 31/415,** A 61 K 31/425 // (A61K31/425, 31:415)

(21) Anmeldenummer: **83810006.3**

(22) Anmeldetag: **07.01.83**

(54) **Anthelmintika.**

(30) Priorität: **13.01.82 CH 179/82**

(43) Veröffentlichungstag der Anmeldung: **27.07.83 Patentblatt 83/30**

(45) Bekanntmachung des Hinweises auf die Patenterteilung: **04.12.85 Patentblatt 85/49**

(84) Benannte Vertragsstaaten: **BE CH DE FR IT LI NL SE**

(56) Entgegenhaltungen:
**EP-A-0 059 074**
**DE-A-2 815 621**

(73) Patentinhaber: **CIBA-GEIGY AG, Postfach, CH-4002 Basel (CH)**

(72) Erfinder: **Boray, Joseph Coleman, Dr., 21, Bogota Avenue, Neutral Bay, N.S.W. 2089 (AU)**

**EP 0 084 516 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue anthelmintische Mittel, die als aktive Komponente eine Wirkstoffkombination enthalten, und ihre Verwendung zur Bekämpfung von Helminthen, insbesondere von Trematoden, in Haus- und Nutztieren.

Die den erfindungsgemäßen Mitteln zugrundeliegenden Wirkstoffkombinationen weisen eine synergistische Wirksamkeit auf, welche die additive Wirkung der kombinierten Einzelwirkstoffe in vorteilhafter Weise übertrifft. Die erfindungsgemäßen Wirkstoffkombinationen bestehen aus folgenden Einzelwirkstoffen:

Einer Verbindung der Formel I

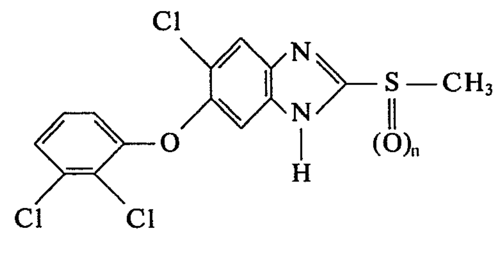

(I)

in welcher n gleich 0 oder 1 bedeutet, und einer Verbindung der Formel II

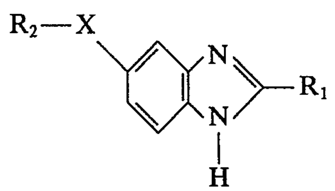

(II)

in welcher $R_1$ die Gruppe —NH—COO—$R_3$, worin $R_3$ einen Alkylrest mit 1 bis 9 Kohlenstoffatomen, vorzugsweise Methyl, darstellt, oder einen Thiazol-4-yl-Rest, $R_2$ einen Alkylrest mit 1 bis 4 Kohlenstoffatomen, vorzugsweise n-Propyl, gegebenenfalls durch Halogen oder Alkyl substituiertes Phenyl oder einen Cycloalkylrest mit 3 bis 6 Kohlenstoffatomen und X O, S, SO, CO, Methylen, vorzugsweise S oder SO, oder einen —NH—COO-Rest bedeuten, einschließlich der unter die Formel II fallenden Isomeren sowie ihrer Säureadditionssalze.

Die als Wirkstoffkomponenten der erfindungsgemäßen Mittel vorstehend beschriebenen Verbindungen sind individuell als anthelmintisch wirksam bekannt. Verbindungen der Formel I sind beispielsweise in der deutschen Offenlegungsschrift 2 815 621 und diejenigen der Formel II in Am. Journ. Vet. Res. 37, 1515—16 (1976), in der belgischen Patentschrift 793 358 und in Vet. Rec. 101, (9) 260—63, 1977 beschrieben. In der genannten Offenlegungsschrift werden im Rahmen einer Aufzählung von Wirkstoffen als Kombinationspartner in allgemeiner Weise Kombinationen von Verbindungen der Formel I mit solchen der Formel II vorgeschlagen, jedoch keine spezifischen Mischungen mit zahlenmäßig umrissenen Gewichtsverhältnissen genannt, wie sie der vorliegenden Erfindung durch das überraschende Auftreten einer synergistischen Wirkungspotenzierung bei bestimmten, später definierten Gewichtsverhältnissen zugrundeliegen.

Die Kombination von verschiedenen chemischen Verbindungen zur Erzeugung synergistischer Wirkungsverstärkung für die Bekämpfung von Schädlingen erlangt durch die damit gegebene Möglichkeit, der immer häufiger in Erscheinung tretenden Resistenz der Parasiten gegenüber Einzelverbindungen wirksam entgegenzutreten, in zunehmendem Maße an Bedeutung. Eine wegen des Auftretens von Resistenzerscheinungen bei den Parasiten notwendig werdende Erhöhung der zum Einsatz gelangenden Wirkstoffmengen kann somit weitgehend vermieden werden. Die sich daraus ergebende Einsparung an Substanzmengen bringt sowohl wirtschaftliche als auch durch Verminderung der Umweltbelastung bedeutsame ökologische Vorteile mit sich. Für die Verabreichung von Anthelmintika an Nutztiere kann das eine Senkung der Applikationsdosis bedeuten, womit gleichzeitig eine Verminderung der Intoxikation der behandelten Tiere verbunden ist, so daß deren Nutzleistung während der Therapie nicht beeinträchtigt wird.

Wurmerkrankungen von Haus- und Nutztieren sind weit verbreitet. Sie führen zur Hemmung des Wachstums und verminderter Nutzleistung der erkrankten Tiere und können sogar deren Tod verursachen. Eine besonders gefährliche Wurmkrankheit stellt die Fasciolose dar, die durch parasitierende Leberegel (beispielsweise Fasciola hepatica, Fasciola gigantica und Fascioloides spp.) hervorgerufen wird und vor allem bei Wiederkäuern, wie Schafen und Rindern, auftritt. Die durch diese Wurmkrankheit hervorgerufenen Schäden können vor allem bei epidemieartigem Auftreten des Wurmbefalls in Viehherden ein beträchtliches Ausmaß annehmen. Bekämpfung und Vorbeugung der Fasciolose gelten deshalb als vordringliche Aufgabe, um derartige, vor allem volkswirtschaftlich ins Gewicht fallende Schäden in der tierischen Produktion zu vermeiden. Trotz zahlreicher bekannter Präparate auf dem Gebiet der Helminthenbekämpfung ist es bisher nicht gelungen, Wirkstoffe zu entwickeln, die neben guten allgemeinen anthelmintischen Eigenschaften eine zufriedenstellende Wirkung gegen adulte und juvenile Fasciolaspecies in Schafen und Rindern bei gleichzeitig niedriger Toxizität aufweisen.

Es wurde nun gefunden, daß die in den erfindungsgemäßen Mitteln enthaltenen Wirkstoffkombinationen, bestehend aus einer Verbindung der Formel I und Verbindungen der Formel II, eine hohe anthelmintische Wirksamkeit entfalten, die sich nicht nur mit breitem Wirkungsspektrum gegen Nemathoden, Cestoden und Trematoden richtet, sondern eine stark ausgeprägte Wirkung gegen Fasciola spp. in Haus- und Nutztieren (Wiederkäuer und Pferde) besitzt. Dabei ist als besonders vorteilhaft hervorzuheben, daß die den erfindungsgemäßen Mitteln zugrundeliegenden Wirkstoffkombinationen bereits bei geringen Dosen totale Wirkung gegen Leberegel im Schaf erzielen, besonders auch gegen juventile Stadien, so daß Nebenwirkungen, die eine Beeinträchtigung der Entwicklung und der Nutzleistung der behandelten Tiere während der Behandlungszeit verursachen könnten, vermieden werden.

Die gegen Fasciola hepatica gerichtete Wirkung der erfindungsgemäßen Wirkstoffkombinationen liegt signifikant über der additiven Wirkung der die Kombinationen bildenden Einzelverbindungen.

Als bevorzugt sind folgende Kombinationen anzusehen:

A) 5-Chlor-6-(2',3'-dichlorphenoxy)-2-methylthiobenzimidazol mit entweder
B) [5-(Propylthio)-1H-benzimidazol-2-yl]-carbaminsäuremethylester (›Albendazol‹), oder
C) [5-(Phenylthio)-1H-benzimidazol-2-yl]-carbaminsäuremethylester (›Fenbendazol‹).

Für die in den erfindungsgemäßen Wirkstoffkombinationen maßgebenden Gewichtsverhältnisse von Verbindungen der Formel I zu Verbindungen der Formel II gilt der Bereich von 1 : 50 bis 50 : 1. Zur Entfaltung der synergistischen Wirkung ist das Kombinationsverhältnis von 1 : 10 bis 10 : 1 als besonders günstig anzusehen.

Versuche an mit Fasciola hepatica infizierten Schafen

36 Schafe wurden artifiziell mit Metacercarien von Fasciola hepatica infiziert. Zu diesem Zweck wurden die Metacercarien in einer wäßrigen 0,4%igen Suspension von Carboxymethylcellulose direkt in den Rumen der Versuchstiere injiziert. Die Tiere wurden dann für die Medikierung in etwa gleichgroße Gruppen und in drei Kontrollgruppen eingeteilt. Nach 4 Wochen wurden die Wirkstoffe in Dosen von 5 bis 10 mg/kg Körpergewicht je nach vorgesehener Kombination an die Versuchstiere verabreicht.

Während des Versuchs wurden die Schafe in Gehegeboxen unter Normalbedingungen gehalten.

Zur Prüfung der Wirkung der einzelnen Wirkstoffkombinationen wurden mit einer Standardtechnik 12 bis 15 Wochen nach Infizierung bzw. 8 bis 11 Wochen nach Medikierung die von den Leberegeln abgelegten Eier im Kot gezählt. Anschließend wurden die Versuchstiere getötet, soweit sie nicht zur Gruppe B und Kontrollgrupe B' gehörten (Tiere Nr. 26–36), und es wurde die Zahl der vorhandenen Leberegel festgestellt.

Die folgenden Resultate wurden erhalten.

Tabelle 1

| Schafe Nr. | Substanz bzw. Gemisch (Dosis mg/kg) | EPG* | | Adulte Egel bei Autopsie | | |
|---|---|---|---|---|---|---|
| | | 14. Woche | 15. Woche | Anzahl | Mittelwert | % Reduktion |
| 1 | A (5,0) | 65 | 125 | 12 | 10,2 | 89,4 |
| 2 | | 60 | 60 | 23 | | |
| 3 | | 0 | 5 | 2 | | |
| 4 | | 10 | 0 | 14 | | |
| 5 | | 0 | 0 | 0 | | |

Fortsetzung

| Schafe Nr. | Substanz bzw. Gemisch (Dosis mg/kg) | EPG* 14. Woche | 15. Woche | Adulte Egel bei Autopsie Anzahl | Mittelwert | % Reduktion |
|---|---|---|---|---|---|---|
| 6 | A + C (5,0 + 5,0) | 5 | 0 | 4 | 1,0 | 99,0 |
| 7 | | 0 | 0 | 0 | | |
| 8 | | 0 | 0 | 0 | | |
| 9 | | 0 | 0 | 0 | | |
| 10 | A + B (5,0 + 4,6) | 0 | 10 | 2 | 0,8 | 99,2 |
| 11 | | 5 | 15 | 2 | | |
| 12 | | 0 | 0 | 0 | | |
| 13 | | 0 | 0 | 0 | | |
| 14 | | 0 | 0 | 0 | | |
| 15 | unbehandelte Kontrolle A′ | 40 | 300 | 78 | 95,8 | |
| 16 | | 700 | 500 | 115 | | |
| 17 | | 510 | 620 | 119 | | |
| 18 | | 150 | 230 | 62 | | |
| 19 | | 920 | 240 | 105 | | |

Tabelle 2

| Schafe Nr. | Substanz (Dosis mg/kg) | EPG* 12. Woche | 13. Woche | Adulte Egel bei Autopsie Anzahl | Mittelwert | % Reduktion |
|---|---|---|---|---|---|---|
| 20 | C (5,0) | – | – | 54 | 71 | 0 |
| 21 | | | | 77 | | |
| 22 | | | | 82 | | |
| 23 | unbehandelte Kontrolle C′ | – | – | 58 | 70 | |
| 24 | | | | 70 | | |
| 25 | | | | 82 | | |

Fortsetzung

| Schafe Nr. | Substanz (Dosis mg/kg) | EPG* | | Adulte Egel bei Autopsie | | |
|---|---|---|---|---|---|---|
| | | 12. Woche | 13. Woche | Anzahl | Mittelwert | % Reduktion |
| 26 | B (4,6) | 360 | 1110 | – | – | –** |
| 27 | | 370 | 560 | | | |
| 28 | | 290 | 370 | | | |
| 29 | | 880 | 1280 | | | |
| 30 | unbehandelte Kontrolle B' | 710 | 680 | – | – | |
| 31 | | 400 | 670 | | | |
| 32 | | 1290 | 1270 | | | |
| 33 | | 580 | 660 | | | |
| 34 | | 860 | 850 | | | |
| 35 | | 570 | 410 | | | |
| 36 | | 600 | 1450 | | | |

Legende (Tabellen 1 + 2)

Substanz A: 5-Chlor-6-(2',3'-dichlorphenoxy)-2-methylthiobenzimidazol
Substanz C: [5-(Phenylthio)-1H-benzimidazol-2-yl]-carbaminsäuremethylester (›Fenbendazol‹)
Substanz B: [5-(Propylthio)-1H-benzimidazol-2-yl]-carbaminsäuremethylester (›Albendazol‹)
EPG*: Anzahl Leberegeleier pro gr Faeces (Woche nach erfolgter artifizieller Infektion mit Metazerkarien)
**: Aus der Literatur ist bekannt, daß Albendazol bei vergleichbarer Dosis unwirksam gegen 6wöchige und jüngere Leberegel ist (Austral. Vet. J. 55, 431–432, 1979; Brit. Vet. J. 138, 371–382, 1982).

Ergebnis

Die den erfindungsgemäßen Mitteln zugrundeliegenden Wirkstoffkombinationen zeigten über 99% Wirksamkeit, sogar gegen juvenile Fasciola hepatica. Ihre Wirkung liegt damit signifikant über der additiven Wirkung der die Kombination bildenden Einzelverbindungen.

Bei den therapeutisch wirksamen Dosen der Wirkstoffkombinationen wurden an den Versuchstieren klinisch keine Toxizitätssymptome festgestellt.

Die erfindungsgemäßen Wirkstoffkombinationen werden zur Bekämpfung parasitärer Helminthen bei Haus- und Nutztieren, wie Rindern, Schafen, Ziegen, Pferden, Katzen und Hunden sowie bei der Rotwild-Hege verwendet. Die Kombinationen können den Tieren sowohl als Einzeldosis wie auch wiederholt verabreicht werden, wobei die einzelnen Gaben je nach Tierart vorzugsweise zwischen 0,5 und 100 mg pro kg Körpergewicht betragen. Durch eine protrahierte Verabreichung erzielt man in manchen Fällen eine bessere Wirkung oder man kann mit geringeren Gesamtdosen auskommen. Die Wirkstoffe bzw. die sie enthaltenden Gemische können auch dem Futter oder den Tränken zugesetzt werden. Das Fertigfutter enthält die Wirkstoffkombinationen vorzugsweise in einer Konzentration von 0,005 bis 0,1 Gew.-%. Die Mittel können in Form von Lösungen, Emulsionen,Suspensionen (Drenchs), Pulver, Tabletten, Bolussen oder Kapseln peroral den Tieren verabreicht werden. Zur Bereitung dieser Applikationsformen dienen zum Beispiel übliche feste Trägerstoffe wie Kaolin, Talkum, Bentonit, Kochsalz, Calciumphosphat, Baumwollsaatmehl oder mit den Wirkstoffen nicht reagierende Flüssigkeiten wie Öle und andere, für den tierischen Organismus unschädliche Lösungs- und Verdünnungsmittel. Soweit die physikalischen und toxikologischen Eigenschaften von Lösungen oder Emulsionen dies zulassen, können die Wirkstoffkombinationen den Tieren auch beispielsweise subcutan injiziert oder mittels der

Pour-on-Methode appliziert werden. Ferner ist eine Verabreichung der Wirkstoffe an die Tiere auch mittels Lecksteinen (Salz) oder Molasse-Blöcken möglich.

Liegen die anthelmintischen Mittel in Form von Futterkonzentrat vor, so dienen als Hauptträgerstoffe Futtermaterialien wie beispielsweise Heu, Leistungsfutter, Futtergetreide oder Proteinkonzentrate. Das Futter kann außer den Wirkstoffen noch Zusatzstoffe, Vitamine, Antibiotika, Chemotherapeutika oder andere Pestizide, vornehmlich Bakteriostatika, Fungistatika, Coccidiostatika oder auch Hormonpräparate, Stoffe mit anaboler Wirkung oder andere das Wachstum begünstigende, die Fleischqualität von Schlachttieren beeinflussende oder in anderer Weise für den Organismus nützliche Stoffe enthalten. Auch können sie mit Insektiziden und Akariziden kombiniert werden, wodurch ihre Wirkung verbreitert und an gegebene Umstände angepaßt wird.

Die Herstellung der erfindungsgemäßen anthelmintischen Mittel erfolgt in an sich bekannter Weise durch inniges Vermischen und Vermahlen der Wirkstoffkombinationen, bestehend aus Verbindungen der Formel I und Verbindungen der Formel II mit geeigneten Trägerstoffen, gegebenenfalls unter Zusatz von gegenüber den Wirkstoffen inerten Dispersions- oder Lösungsmitteln.

Die erfindungsgemäßen anthelmintischen Mittel können beispielsweise in folgenden Zubereitungsformen angewendet werden:

Feste Zubereitungsformen:

Granulate, Umhüllungsgranulate, Imprägnierungsgranulate und Homogengranulate. In Wasser dispergierbare Wirkstoffkonzentrate (Wettable Powder).

Flüssige Zubereitungsformen:

Lösungen, Pasten, Emulsionen (insbesondere gebrauchsfertige Suspensionen (Drenches).

Die Korngröße der Trägerstoffe beträgt für Stäubemittel und Spritzpulver zweckmäßig bis ca. 0,1 mm und für Granulate 0,01—0,5 mm.

Die Konzentration der Wirkstoffkombinationen in den festen Zubereitungsformen beträgt 0,5 bis 80%, in den flüssigen Aufarbeitungsformen 0,5 bis 50%.

Diesen Gemischen können ferner die Wirkstoffkombinationen stabilisierende Zusätze und/oder nichtionische, anionaktive und kationaktive Stoffe zugegeben werden, die beispielsweise eine bessere Benetzbarkeit(Netzmittel) sowie Dispergierbarkeit (Dispergatoren) gewährleisten.

Beispiel

In Wasser dispergierbare Pulvermischung

| | |
|---|---|
| 25 Gew.-Teile | Wirkstoffkombination von Verbindungen der Formel I mit Verbindungen der Formel II werden in einem Mischapparat mit |
| 7,5 Gew.-Teilen | eines aufsaugenden Trägermaterials beispielsweise Kieselsäure und |
| 59,4 Gew.-Teilen | eines Trägermaterials beispielsweise Bolus alba oder Kaolin und |
| 0,5 Gew.-Teilen | Ölsäure und |
| 5,3 Gew.-Teilen | Octylphenylpolyglykoläther |
| 2,3 Gew.-Teilen | Stearyl-benzimidazol-Derivate intensiv vermischt. |

Diese Mischung wird auf einer Stift- oder Luftstrahlmühle bis zu einer Partikalgröße von 5—15 μm gemahlen. Das so erhaltene Spritzpulver gibt in Wasser eine gute Suspension.

**Patentansprüche**

1. Mittel zur Bekämpfung parasitärer Helminthen, dadurch gekennzeichnet, daß es als aktive Komponente eine Wirkstoffkombination bestehend aus mindestens einer Verbindung der Formel I

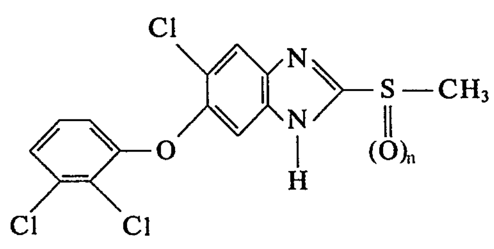

(I)

in welcher n gleich 0 oder 1 bedeutet, und einer Verbindung der Formel II

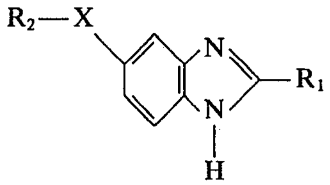

(II)

in welcher $R_1$ die Gruppe –NH–COO–$R_3$, worin $R_3$ einen Alkylrest mit 1 bis 9 Kohlenstoffatomen, vorzugsweise Methyl, darstellt, oder einen Thiazol-4-yl-Rest, $R_2$ einen Alkylrest mit 1 bis 4 Kohlenstoffatomen, vorzugsweise n-Propyl, gegebenenfalls durch Halogen oder Alkyl substituiertes Phenyl oder einen Cycloalkylrest mit 3 bis 6 Kohlenstoffatomen und X O, S, SO, CO, Methylen, vorzugsweise S oder SO, oder einen –NH–COO-Rest bedeuten, einschließlich der unter die Formel II fallenden Isomeren sowie ihrer Säureadditionssalze im Gewichtsverhältnis der Kombinationspartner von 1 : 50 bis 50 : 1 zusammen mit geeigneten Trägerstoffen und/oder Verteilungs- und Verdünnungsmitteln enthält.

2. Mittel zur Bekämpfung parasitärer Helminthen gemäß Anspruch 1, dadurch gekennzeichnet, daß es als aktive Komponente eine Wirkstoffkombination bestehend aus

5-Chlor-6-(2',3'-dichlorphenoxy)-2-methylthiobenzimidazol und
[5-(Propylthio)-1H-benzimidazol-2-yl]-carbaminsäuremethylester

zusammen mit geeigneten Trägerstoffen und/oder Verteilungs- und Verdünnungsmitteln enthält.

3. Mittel zur Bekämpfung parasitärer Helminthen gemäß Anspruch 1, dadurch gekennzeichnet, daß es als aktive Komponente eine Wirkstoffkombination bestehend aus

5-Chlor-6-(2',3'-dichlorphenoxy)-2-methylthiobenzimidazol und
[5-(Phenylthio)-1H-benzimidazol-2-yl]-carbaminsäuremethylester

zusammen mit geeigneten Trägerstoffen und/oder Verteilungs- und Verdünnungsmitteln enthält.

4. Anthelmintische Mittel nach den Ansprüchen 1 bis 3, dadurch gekennzeichnet, daß in der Wirkstoffkombination das Gewichtsverhältnis von Verbindungen der Formel I zu Verbindungen der Formel II 1 : 10 bis 10 : 1 beträgt.

5. Mittel gemäß den Ansprüchen 1 bis 4 zur Behandlung von Haus- und Nutztieren.

6. Verwendung von Wirkstoffkombinationen gemäß den Ansprüchen 1 bis 4 zur Herstellung anthelmintischer Präparate für die Bekämpfung von parasitären Helminthen.

7. Verwendung von Wirkstoffkombinationen gemäß den Ansprüchen 1 bis 4 zur Herstellung anthelmintischer Präparate für die Bekämpfung von Trematoden.

8. Mittel gemäß den Ansprüchen 1 bis 5 zur Bekämpfung von Leberegeln (Fasciola spp.).

9. Verwendung von Wirkstoffkombinationen gemäß den Ansprüchen 1 bis 4 zur Herstellung anthelmintischer Präparate für die Bekämpfung von juvenilen und adulten Leberegeln.

## Claims

1. A composition for controlling parasitic helminths, which composition contains as active component an activesubstance combination comprising at least one compound of the formula I

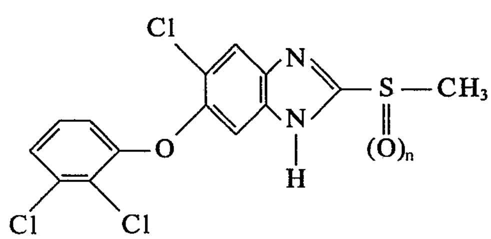

(I)

wherein n is 0 or 1, and at least one compound of the formula II

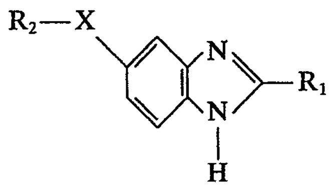

(II)

wherein $R_1$ is the group $-NH-COO-R_3$, in which $R_3$ is an alkyl group having 1 to 9 carbon atoms, preferably methyl, or $R_1$ is a thiazol-4-yl group, $R_2$ is an alkyl group having 1 to 4 carbon atoms, preferably n-propyl, phenyl unsubstituted or substituted by halogen or alkyl, or it is a cycloalkyl group having 3 to 6 carbon atoms, and X is O, S, SO, CO, methylene, preferably S or SO, or an $-NH-COO$ group, or an isomer embraced by the formula II or an acid addition salt of such a compound, the weight ratio of the combination partners being from 1 : 50 to 50 : 1, together with suitable carriers and/or dispersing agents and diluents.

2. A composition for controlling parasitic helminths, according to claim 1, which contains as active component an active-substance comprising:

5-chloro-6-(2',3'-dichlorophenoxy)-2-methylthiobenzimidazole and
[5-(propylthio)-1H-benzimidazol-2-yl]carbamic acid methyl ester,

together with suitable carriers and/or dispersing agents and diluents.

3. A composition for controlling parasitic helminths, according to claim 1, which contains as active component an active-substance combination comprising:

5-chloro-6-(2',3'-dichlorophenoxy)-2-methylthiobenzimidazole and
[5-(phenylthio)-1H-benzimidazol-2-yl]carbamic acid methyl ester,

together with suitable carriers and/or dispersing agents and diluents.

4. An anthelmintic composition according to any one of claims 1 to 3, wherein the weight ratio, in the active-substance combination, of compounds of the formula I to compounds of the formula II is 1 : 10 to 10 : 1.

5. A composition according to any one of claims 1 to 4 for the treatment of domestic animals and productive livestock.

6. Use of an active-substance combination according to any one of claims 1 to 4 for the preparation of an anthelmintic composition for controlling parasitic helminths.

7. Use of an active-substance combination according to any one of claims 1 to 4 for the preparation of an anthelmintic composition for controlling trematodes.

8. A composition according to any one of claims 1 to 5 for controlling liver flukes (Flasciola spp.).

9. Use of an active-substance combination according to any one of claims 1 to 4 for the preparation of an anthelmintic composition for controlling juvenile and adult liver flukes.

## Revendications

1. Produit pour combattre les helminthes parasitaires, caractérisé en ce qu'il contient en tant que composant actif une combinaison de substances actives consistant en au moins un composé de formule I

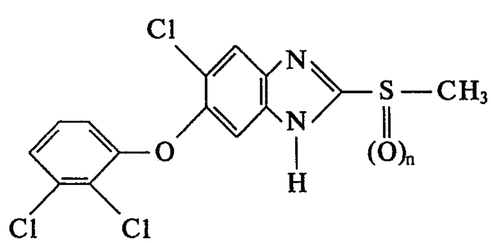

(I)

dans laquelle n est égal à 0 ou 1,
et au moins un composé de formule II

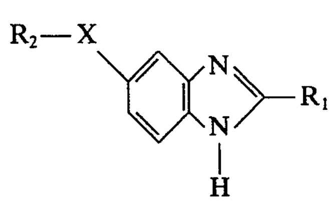

(II)

dans laquelle $R_1$ représente le groupe $-NH-COO-R_3$ dans lequel $R_3$ est un groupe alkyle en C 1—C 9, de préférence un groupe méthyle, ou un reste thiazole-4-yle, R 2 représente un groupe alkyle en C 1—C 4, de préférence un groupe n-propyle, un groupe phényle éventuellement substitué par des halogènes ou des groupes alkyle, ou un reste cycloalkyle contenant 3 à 6 atomes de carbone et X représente O, S, SO, CO, un groupe méthylène, de préférence S ou SO, ou un reste —NH—COO—, y compris les isomères répondant à la formule II, et leurs sels formés par addition avec des acides, dans des proportions relatives en poids des composants de la combinaison allant de 1 : 50 à 50 : 1, avec des véhicules et/ou des diluants solides et liquides appropriés.

2. Produit pour combattre les helminthes parasitaires selon la revendication 1, caractérisé en ce qu'il contient en tant que composant actif une combinaison de substances actives consistant en:

le 5-chloro-6-(2',3'-dichlorophénoxy)-2-méthylthiobenzimidazole, et
le [5-(propylthio)-1H-benzimidazole-2-yl]-carbamate de méthyle,

avec des véhicules et/ou des diluants solides et liquides appropriés.

3. Produit pour combattre les helminthes parasitaires selon la revendication 1, caractérisé en ce qu'il contient en tant que composant actif une combinaison de substances actives consistant en:

le 5-chloro-6-(2',3'-dichlorophénoxy)-2-méthylthiobenzimidazole et
le [5-(phénylthio)-1H-benzimidazole-2-yl]-carbamate de méthyle,

avec des véhicules et/ou des diluants solides et liquides appropriés.

4. Produit anthelminthique selon les revendications 1 à 3, caractérisé en ce que, dans la combinaison de substances actives, les proportions relatives en poids entre les composés de formule I et les composés de formule II vont de 1 : 10 à 10 : 1.

5. Produit selon les revendications 1 à 4, pour le traitement des animaux familiers et utiles.

6. Utilisation des combinaisons de substances actives selon les revendications 1 à 4, pour la préparation de produits anthelminthiques servant à combattre les helminthes parasitaires.

7. Utilisation des combinaisons de substances actives selon les revendications 1 à 4, pour la préparation de produits anthelminthiques servant à combattre les trématodes.

8. Produit selon les revendications 1 à 5, pour combattre les douves (Fasciola spp.).

9. Utilisation des combinaisons de substances actives selon les revendications 1 à 4, pour la préparation de produits anthelminthiques servant à combattre les douves jeunes et adultes.